# EUROPEAN PATENT SPECIFICATION

(11) **EP 2 152 910 B1**
(45) Date of publication and mention of the grant of the patent: **14.09.2011**
(21) Application number: 08747528.1
(22) Date of filing: 02.05.2008
(51) Int. Cl.: C12Q 1/68

(54) **DETECTING TRIAZOLE RESISTANCE IN ASPERGILLUS**
NACHWEIS VON TRIAZOLRESISTENZ BEI ASPERGILLUS
DÉTECTION DE LA RÉSISTANCE AU TRIAZOLE DANS L'ASPERGILLUS

(30) Priority: 04.05.2007 US 916193 P
(43) Date of publication of application: 17.02.2010
(73) Proprietor: Myconostica Limited, Sharston Manchester M22 4SN (GB)
(72) Inventor: ROCHA, Eleusa Maria, F., New York, NY 11103 (US); PARK, Steven, Whitestone, NY 11357 (US); PERLIN, David, S., Chappaqua, NY 10514 (US)
(74) Representative: Swinkels, Bart Willem
(86) International application number: PCT/US2008/062463
(87) International publication number: WO 2008/137715

(56) References cited:
- COSTE ALIX ET AL: "A mutation in Tac1p, a transcription factor regulating CDR1 and CDR2, is coupled with loss of heterozygosity at chromosome 5 to mediate antifungal resistance in Candida albicans" GENETICS, vol. 172, no. 4, April 2006 (2006-04), pages 2139-2156, XP002496769 ISSN: 0016-6731
- MELLADO E ET AL: "New resistance mechanisms to azole drugs in Aspergillus fumigatus and emergence of antifungal drugs-resistant A-fumigatus atypical strains" MEDICAL MYCOLOGY, vol. 44, no. Suppl. 1, September 2006 (2006-09), pages S367-S371, XP008096705 ISSN: 1369-3786
- NIERMAN WILLIAM C ET AL: "Genomic sequence of the pathogenic and allergenic filamentous fungus Aspergillus fumigatus (vol 438, pg 1151, 2005)" NATURE (LONDON), vol. 439, no. 7075, January 2006 (2006-01), page 502, XP002496771 ISSN: 0028-0836 & DATABASE GENBANK 27 February 2008 (2008-02-27), "Aspergillus fumigatus Af293 C6 transcription factor (REF SEQ of XP_753953)" retrieved from NCBI Database accession no. xm_748860 & DATABASE GENPEPT 27 February 2008 (2008-02-27), "C6 transcription factor Aspergillus fumigatus Af293" retrieved from NCBI Database accession no. XP_753953

## Description

### CROSS-REFERENCE TO RELATED APPLICATIONS

This application claims priority to U.S. Provisional Application No. 60/916,193, filed May 4, 2007.

### BACKGROUND

Fungal infections are a significant cause of morbidity and mortality in a variety of severely ill patients. For instance, fungi can cause superficial and often fatal disseminated infections in immunocompromised patients. Systemic fungal infections cause approximately 25% of infection-related deaths in leukaemics and 5-10% of deaths in patients undergoing lung, pancreas or liver transplantation. Acquired fungal sepsis also is known to occur in up to 13% of very low birth-weight infants. Members of the *Aspergillus* genus are the second most common cause of fungal infections behind the members of the *Candida* genus.

Fungal infections caused by members of the *Aspergillus* genus commonly are treated with triazoles. Triazoles act by blocking the ergosterol biosynthetic pathway at the C-14 demethylation stage. Chamilos et al. Drug Resistance Updates (2005) 8:344-358. Triazoles include, for example, voriconazole, isavuconazole, ravuconazole, itraconazole and posaconazole. While triazoles have been effective in treating *Aspergillus* fungal infections, there has been an increase in resistance of *Aspergillus* to triazole treatment. Chamilose *et al., supra.* Such resistance can render triazole treatment ineffective.

Recognizing whether a particular fungal infection comprises triazole-resistant *Aspergillus* is important in providing appropriate care to patients. Thus, methods are needed for determining whether a particular *Aspergillus* fungus is susceptible to triazole treatment.

### SUMMARY

In one aspect, methods are provided for detecting triazole-resistant fungi in a sample comprising evaluating the sample for the presence of a gene encoding a mutant AzRF1 transcription factor, wherein the mutant AzRF1 contains a deletion of residues QSQS at position 559-562 of said gene and correlating the presence of the mutant gene to the presence of the triazole-resistant fungus. In some aspects, the evaluation involves measuring the expression level of said gene. In some embodiments, the evaluation comprises contacting said sample with an oligonucleotide capable of hybridizing to said mutant gene. The oligonucleotide can comprise a detectable label. In one example, the oligonucleotide comprises a fluorophore and a chromophore, while in another, the oligonucleotide comprises a fluorophore and a non-fluorescent quencher.

In some embodiments, the oligonucleotide comprises the nucleic acid sequence of SEQ ID NO: 5 or SEQ ID NO: 6. In one embodiment, the oligonucleotide can comprise greater than 90% homology to the nucleic acid sequence of SEQ ID NO: 5 or SEQ ID NO: 6. In another, the oligonucleotide can comprise the nucleic acid sequence of SEQ ID NO: 7, SEQ ID NO: 8 or SEQ ID NO: 9.

In some aspects, the mutant gene is amplified prior to said hybridization. The amplification can comprise PCR. The amplification also can comprise contacting the sample with primers having the nucleic acid sequence of SEQ ID NO: 3 and SEQ ID NO: 4.

In other embodiments, the triazole-resistant fungus belongs to the genus *Aspergillus.* The triazole-resistant fungus can be *Aspergillus fumigatus.*

In some cases, the triazole-resistant fungus is resistant to itraconazole, posaconazole, voriconazole, isavuconazole, or ravuconazole.

In another embodiment, a method of detecting triazole-resistant fungi in a sample comprises (A) evaluating the sample for the presence of a gene encoding a AzRF1 transcription factor and (B) correlating the presence of the gene to the presence of the triazole-resistant fungus. In some aspects, the evaluation involves measuring the expression level of the gene.

In another aspect, a kit is provided for detecting triazole-resistant fungi in a sample comprising an oligonucleotide having the nucleic acid sequence of SEQ ID NO: 5 or SEQ ID NO: 6 or a sequence with greater than 90% homology to SEQ ID NO: 5 or SEQ ID NO: 6. In some embodiments the oligonucleotide comprises a label and the kit further comprises one or more amplification primers.

Other objects, features and advantages will become apparent from the following detailed description. The detailed description and specific examples are given for illustration. Further, the examples demonstrate the principle of the invention and cannot be expected to specifically illustrate the application of this invention to all the examples where it will be obviously useful to those skilled in the prior art.

### BRIEF DESCRIPTION OF THE FIGURES

Figure 1 shows a sequence alignment of the GAL4-like Zn2Cys6 binuclear cluster DNA-binding domain region of the 8 putative transcription factors in A. *fumigatus.*

Figure 2 shows an amino acid sequence alignment of wild and mutant AzRF1 transcription factors.

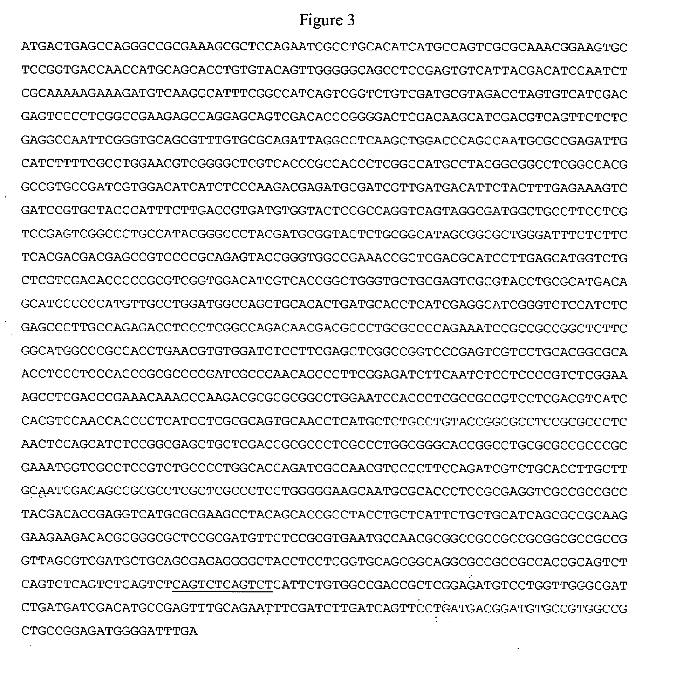

Figure 3 provides a partial mRNA sequence (SEQ ID NO: 1) of the C6 transcription factor (AzRF1) of *Aspergillus fumigatus* Af293 (AFUA_5G06800) as noted in GeneBank at gi|70998461|ref|XM_748860.1.

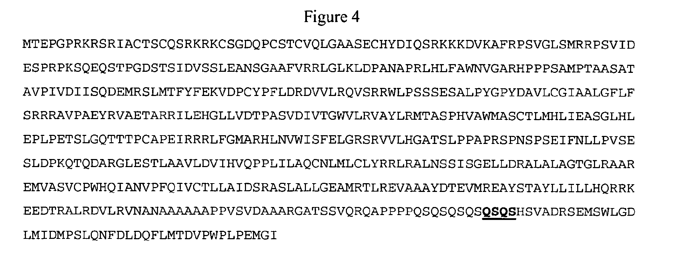

Figure 4 provides the amino acid sequence (SEQ ID NO: 2) of the C6 transcription factor (AzRF1) of *Aspergillus fumigatus* Af293, as noted in GeneBank at gi|70998462|ref|XP_753953.1|.

### DESCRIPTION OF THE SEQUENCES

SEQ ID NO: 3, 5'-CGGTTAGCGTCGATGCTGC-3', is an exemplarly forward primer for amplification reactions.

SEQ ID NO: 4, 5'-AGGACATCTCCGAGCGGTC-3', is an exemplarily reverse primer for amplification reactions.

SEQ ID NO: 5, 5'-CCGCAGTCTCAGTCTCAGTCTCAGTCTCAT-3', is an exemplarily detection probe.

SEQ ID NO: 6,5'-ACCGCAGTCTCAGTCTCAGTCTCAGTCTCATT-3', is another exemplarily detection probe.

SEQ ID NO: 7,5'-CGGCAGCCCGCAGTCTCAGTCTCAGTCTCAGTCTCATGCTGCCG-3', is an exemplarily detection probe.

SEQ ID NO: 8, 5'-CGGCGGCACCGCAGTCTCAGTCTCAGTCTCAGTCTCATTGCCGCCG-3', is another exemplarily detection probe.

SEQ ID NO: 9, 5'-CGGCGGCCCGCAGTCTCAGTCTCAGTCTCAGTCTCATGCCGCCG-3', is another exemplarily detection probe.

### DETAILED DESCRIPTION

The inventors have discovered regions of DNA specific to mutant fungi that are resistant to triazole drugs. Accordingly, these DNA regions can be used to determine whether a sample contains triazole-resistant fungi. Methods of detecting triazole-resistant fungi, therefore, are provided, as are novel probes and primers that can be used to detect these regions.

### Detecting Triazole-Resistance

In one aspect, methods are provided for detecting triazole-resistant fungi in a sample comprising evaluating the sample for the presence of a gene encoding a mutant AzRF1 transcription factor. In one embodiment, the methods comprise evaluating the sample for the presence of a gene encoding a mutant AzRF1 transcription factor, wherein the mutant AzRF1 contains a deletion of residues QSQS at position 559-562 of the gene and correlating the presence of the mutant gene to the presence of the triazole-resistant fungus.

In another embodiment, triazole resistance can be assessed in fungi by measuring the activity of the AzRF1 gene in the fungi. In particular, triazole-resistant fungi have been found to possess abnormal amounts of AzRF1 transcription factor. Thus, measuring the copy number of the AzRF1 gene or its expression levels can be useful for detecting triazole-resistance. In some aspects, such detection methods involve ascertaining whether the activity of the AzRF1 gene in the test fungal strain is 2 fold, 3 fold, 4 fold, 5 fold, 6 fold, 7 fold or more higher than typical levels.

Samples can be obtained from biological or non-biological sources. Examples of biological sources include, but are not limited to, biological fluid, tissue, or a combination of thereof. Examples of non-biological sources include, but are not limited to, samples obtained from the environment, such as an air sample, a water sample, a soil sample, or combinations thereof. Other examples of non-biological sources are a piece of a vehicle, watercraft, aircraft, building or dwelling.

In one embodiment, methods are provided for the rapid detection of the presence or absence in a sample of a triazole-resistant fungus belonging to the genus *Aspergillus.* The methods involve detecting in the sample a region of fungal DNA that is specific for triazole-resistant *Aspergillus.* The presence in a sample of a region of fungal DNA that is specific for a particular triazole-resistant genus is indicative of the presence of a fungus belonging to that type of genus. The absence from the sample of a region of fungal DNA that is specific for a particular triazole-resistant genus is indicative of the absence of a fungus belonging to that type of genus.

Methods for detecting the presence of a triazole-resistant fungus belonging to the genera *Aspergillus* can be carried out on any sample. Specific types of sample are discussed in more detail below. In one embodiment, the methods are carried out on a sample that is known to contain a fungus. For instance, the methods can be carried out on a sample that has already undergone a panfungal detection method and a positive result was achieved. The methods also can be carried out on a sample to confirm the identity of one or more triazole-resistant fungi whose presence in the sample is known. In another embodiment, the methods are carried on a sample whose fungus-containing status is not known.

In some embodiments, the detection methods can be used to detect the presence or absence of any triazole-resistant species of fungus belonging to the genus *Aspergillus.* For example, the fungus can be *Aspergillus alliaceus, Aspergillus alutaceus, Aspergillus atroviolaceus, Aspergillus caesiellus, Aspergillus candidus, Aspergillus carneus, Aspergillus chevalieri, Aspergillus clavato-nanicus, Aspergillus clavatus, Aspergillus conicus, Aspergillus deflectus, Aspergillus fischerianus, Aspergillus flavipes, Aspergillus flavus, Aspergillus fumigatus, Aspergillus glaucus, Aspergillus hollandicus, Aspergillus janus, Aspergillus japonicus, Aspergillus nidulans, Aspergillus niger, Aspergillus niger var. awamorii, Aspergillus niveus, Aspergillus ochraceus, Aspergillus oryzae, Aspergillus penicilloides, Aspergillus reptans, Aspergillus restrictus, Aspergillus rubrobrunneus, Aspergillus spinosus, Aspergillus sydowii, Aspergillus tamarii, Aspergillus terreus, Aspergillus tetrazonus, Aspergillus unguis, Aspergillus ustus or Aspergillus versicolor.*

In one embodiment, the fungal region of DNA that is specific for triazole-resistant *Aspergillus* ("the fungal region") is itself detected. In another embodiment, any RNA transcribed from the DNA fungal region is detected.

In one embodiment, only the fungal region is detected. In another embodiment, the fungal region is detected as part of larger sequence. For instance, the region can be detected as part of a sequence that has flanking sequences.

The fungal region can be detected using any method known in the art. The fungal region is preferably detected using a probe that specifically hybridizes to the region. In one aspect, the detection comprises contacting the probe with the sample under conditions in which the probe specifically hybridizes to the region, if present, and determining the presence or absence of the hybridization product. The presence of the hybridization product indicates the presence of the fungal region. Conversely, the absence of the hybridization product indicates the absence of the fungal region.

The probe is typically a nucleic acid, such as DNA, RNA, PNA or a synthetic nucleic acid. A probe specifically hybridizes to the fungal regions if it preferentially or selectively hybridizes to the fungal region and not to other DNA or RNA sequences.

In one embodiment, a probe specifically hybridizes to the fungal region under stringent conditions. Hybridization conditions of various stringencies are well-known in the art (for example, Sambrook et al., 2001, Molecular Cloning: a laboratory manual, 3rd edition, Cold Spring Harbour Laboratory Press; and Current Protocols in Molecular Biology, Chapter 2; Ausubel et al., Eds., Greene Publishing and Wiley-lnterscience, New York (1995)). Detection can be carried out under low stringency conditions, for example in the presence of a buffered solution of 30 to 35% formamide, 1 M Nacl and 1 % SDS (sodium dodecyl sulfate) at 37°C followed by a wash in from 1X (0.1650 M Na⁺) to 2X (0.33 M Na⁺) SSC (standard sodium citrate) at 50°C. Detection can be carried out under moderate stringency conditions, for example in the presence of a buffer solution of 40 to 45% formamide, 1 M NaCl, and 1 % SDS at 37°C, followed by a wash in from 0.5X (0.0825 M Na⁺) to 1X (0.1650 M Na⁺) SSC at 55°C. Detection can be carried out under high stringency conditions, for example in the presence of a buffered solution of 50% formamide, 1 M NaCl, 1% SDS at 37°C, followed by a wash in 0.1 X (0.0165 M Na⁺) SSC at 60°C.

The probe can be the same length as, shorter than or longer than the fungal region. The probe is typically at least 5, at least 6, at least 7, at least 8, at least 9, at least 10, at least 15, at least 20, at least 25, at least 30, at least 35, at least 45, at least 50, at least 75 or at least 100 nucleotides in length. For example, the probe can be from 5 to 200, from 7 to 100, from 10 to 50 nucleotides in length. The probe is preferably 5, 10, 15, 20, 25, 30, 35 or 40 nucleotides in length. The probe preferably includes a sequence that shares at least 80%, at least 85%, at least 90%, at least 95%, at least 98%, at least 99% homology based on sequence identity with the fungal region. Homology can be determined as discussed above.

In one embodiment, the probe is detectably-labeled. Any detectable label can be used. Suitable labels include, but are not limited to, fluorescent molecules, radioisotopes, e.g. ¹²⁵I, ³⁵S, enzymes, antibodies and linkers, such as biotin.

The probe can be a molecular beacon probe. *See, e.g.* U.S. Pat. No. 6,150,097. Molecular beacon probes comprise a fluroescent label at one end and a quenching molecule at the other. In the absence of the region to be detected, the probe forms a hairpin loop and the quenching molecule is brought into close proximity with the fluorescent label so that no signal can be detected. Upon hybridization of the probe to the region to be detected, the loop unzips and the fluorescent molecule is separated from the quencher such that a signal can be detected. Suitable fluorescent molecule and quencher combinations for use in molecular beacons are known in the art. Such combinations include, but are not limited to, carboxyfluorsecein (FAM) and dabcyl.

In one embodiment, the probe can be immobilized on a support using any technology which is known in the art. Suitable solid supports are well-known and include plates, such as multi well plates, filters, membranes, beads, chips, pins, dipsticks and porous carriers.

In one aspect, detection of the fungal region comprises amplifying the fungal region or the RNA transcribed therefrom. In one embodiment, the region is amplified before its presence is determined. In another embodiment, the region is detected in real-time as its presence is determined. Real-time methods are disclosed in the Examples and have been described in the art. Such methods are described in, for example, U.S. Patent Nos. 5,487,972 and 6,214,979 and Afonia et al. (Biotechniques, 2002; 32: 946-9), all of which are hereby incorporated by reference.

In one embodiment, only the region to be detected is amplified. In other embodiments, the region to be detected is amplified as part of a much larger length of fungal DNA or RNA. Sequences of DNA or RNA having at least 10, at least 20, at least 30, at least 40, at least 50, at least 60, at least 70, at least 80, at least 90, at least 100, at least 150, at least 200, at least 250, at least 300, at least 400 or at least 500 nucleotides and comprising the region to be detected can be amplified. For example, sequences having from 10 to 2000, from 20 to 1500, from 50 to 1000 or from 100 to 500 nucleotides can be amplified. Such sequences may be located upstream of the target gene for triazole antifungal drugs (14 alpha demethylase otherwise known as *Cyp51A* or *Cyp 51B*) and control expression of said genes.

The DNA or RNA can be amplified using routine methods that are known in the art. In some embodiments, the amplification of fungal DNA is carried out using polymerase chain reaction (PCR) (*See, e.g.* U.S. Pat. Nos. 4,683,195 and 4,683,202); ligase chain reaction ("LCR") (*See, e.g.* Landegren et al., Science 241:1077-1080 (1988); D.Y. Wu and R.B. Wallace, Genomics 4:560-569 (1989); and F. Barany, PCR Methods Appl. 1:5-16 (1991)); loop-mediated isothermal amplification ("LAMP") (Nagamin et al., Clin. Chem. 47(9):1742-1743 (2001); Notomi et al., Nucleic Acids Res. 28(12):E63 (2000)); nucleic acid sequence based analysis (NASBA)( J. Compton, Nature 350:91-92 (1991)); self-sustained sequence replication ("3SR")( Guatelli et al., Proc. Natl. Acad. Sci. U.S.A. 87(5):1874-1878 (1990)); strand displacement amplification ("SDA") (Walker et al., Nucleic Acids Res., 20:1691-1696 (1992); and Walker et al., Proc. Natl. Acad. Sci. U.S.A. 89:392-396 (1992)); or transcription mediated amplification ("TMA") (Pasternack et al., J. Clin. Microbiol. 35(3):676-678 (1997)).

A person skilled in the art can readily design specific primers to amplify a nucleic acid comprising the region of the discovered deletion and the AzRF1 transcription factor generally. Primers are normally designed to be complementary to sequences at either end of the sequence to be amplified but not complementary to any other sequences. Primer design is discussed in, for example, Sambrook *et al.,* 2001, *supra.*

Amplicons can be detected using any method known in the art, including those described above. In one embodiment, an hydrolysis probe format (*e.g*., TAQMAN) with Minor Groove Binder (MGB) moiety can be used to detect amplicons. In another embodiment, a cyanine dye that binds to double-stranded DNA can be used. Exemplary cyanine dyes include, but are not limited to, SYBR GREEN II, SYBR GOLD, YO (Oxazole Yellow), TO (Thiazole Orange), and PG (PicoGreen).

In other embodiments, the testing step can comprise conducting a melting curve analysis. Inspection of fluorescence-versus-temperature plots at the end of PCR can provide additional information when certain dyes or probe formats are used. For example, with the dye SYBR Green, the purity and identity of the PCR products can be confirmed through their melting temperatures. Similarly, when hybridization probes are used, sequence alterations, including polymorphisms, can be distinguished by probe melting temperature.

In one example, immediately after the last PCR cycle, the samples are denatured at 90°C~95°C, cooled to about 5°C~10°C below the Tₘ range of interest and then slowly heated at a ramp rate typically ranging from 0.1 to 0.4°C/sec, while fluorescence is continuously monitored. A notable decrease in fluorescence is observed when a temperature is reached at which, depending on the particular fluorescence chemistry, either (a) a probe dissociates from the amplicon (in the case of hybridization probes) or (b) the double-stranded PCR product dissociates into single-stranded DNA.

The melting transition does not occur all at once but takes place over a small range of temperatures. The middle of the melting curve slope on the fluorescence-versus-temperature plot is referred to as the Tₘ. The melting temperature or Tm is a measure of the thermal stability of a DNA duplex and is dependent on numerous factors, including the length, G/C content and relative position of each type of nucleotide (A,T,G,C, etc.) (Wetmur, J.G. 1997. DNA Probes: applications of the principles of nucleic acid hybridization. Crit Rev Biochem Mol Biol. 26:227-259). The melting temperature is further dependent upon the number, relative position, and type of nucleotide mismatches (A:A, A:G, G:T, G:A, etc), which may occur between DNA:DNA or Probe:DNA duplexes (S.H. Ke and Wartell, R. 1993. Influence of nearest neighbor sequence on the stability of base pair mismatches in long DNA: determination by temperature-gradient gel electrophoresis. Nucleic Acids Res 21:5137-5143.) It is therefore possible to confirm the presence of a particular amplicon by melting temperature if the size and sequence of the target product is known. Likewise, it is possible to differentiate two distinct species on the basis of differential melting temperature due to sequence variation. The practicality and usefulness of melting curve analysis in PCR-based detection systems is well known.

In one embodiment, the region of fungal DNA that is specific for triazole-resistance in *Aspergillus* is detected using a molecular beacon probe selected from the group consisting of SEQ ID NO: 7, SEQ ID NO: 8 and SEQ ID NO: 9. In another embodiment, the region of fungal DNA responsible for triazole-resistance in *Aspergillus* is amplified using the primers of SEQ ID NOs: 3 and 4.

In some embodiments, the detection methods of the invention further comprise an internal amplification control.

### Samples

Any suitable sample can be used. In one embodiment, a biological sample is used. The biological sample can be obtained from or extracted from any organism.

In one aspect, a fluid sample is used, such as a body fluid. Exemplary samples include, but are not limited to, urine, lymph, saliva, cerebrospinal fluid, peritoneal fluid, pericardial fluid, vitreous or other ocular sample, plural fluid, vaginal fluid, mucus, pus or amniotic fluid but is preferably blood, plasma and serum. The sample can be a cell or tissue sample, such as lung, brain, liver, skin or nails.

In one example, a sample is human in origin, while in another non-human samples are utilized. For instance, the sample can be from commercially-farmed animals such as horses, cattle, sheep or pigs or from pets such as cats or dogs. Samples from plants also can be evaluated.

The methods also can be performed on non-biological samples. The non-biological sample can be a fluid or a solid. Examples of non-biological samples include, but are not limited to, surgical fluids, air, soil, water, such as drinking water, reagents for laboratory tests and household containers. Alternatively, the non-biological sample can be a particle collection device containing air, water, another liquid or material.

In one embodiment, a sample is processed prior to being tested, for example by centrifugation or by passage through a membrane that filters out unwanted molecules or cells, such as red blood cells. Alternatively, a sample may be amplified, for example by PCR, prior to testing. In some cases, a sample is tested immediately or soon after isolation, while in others a sample is stored, for example below -70°C, prior to assay.

### Kits

In one aspect, kits are provided for detecting triazole-resistant fungi in a sample. In one embodiment, a kit comprises an oligonucleotide having the nucleic acid sequence of SEQ ID NOS: 5 or 6 or a sequence with greater than 90% homology to SEQ ID NOS: 5 or 6. The oligonucleotide can comprise a label, and the kit can further comprise one or more amplification primers. In another embodiment, the kit comprises a molecular beacon probe having the nucleic acid sequence of SEQ ID NO: 7, SEQ ID NO:8 or SEQ ID NO: 9 and amplification primers having the nucleic acid sequences of SEQ ID NOs: 3 and 4.

In other embodiments, the kits can comprise reagents for extracting fungal DNA or RNA from a sample and/or primers that can be used to amplify the region of fungal DNA and/or an internal control for the amplification and detection stages. The kit additionally can comprise one or more reagents or instruments which enable the inventive methods to be carried out. Such reagents or instruments include, for example, one or more of the following: suitable buffer(s) (aqueous solutions), means to obtain a sample from the subject (such as a vessel or an instrument comprising a needle) or a support comprising wells on which reactions can be done. Reagents can be in a dry state, such that a fluid sample resuspends the reagents. The kit also can comprise instructions for using the reagents to detect the presence of a triazole-resistant fungus.

### Example 1

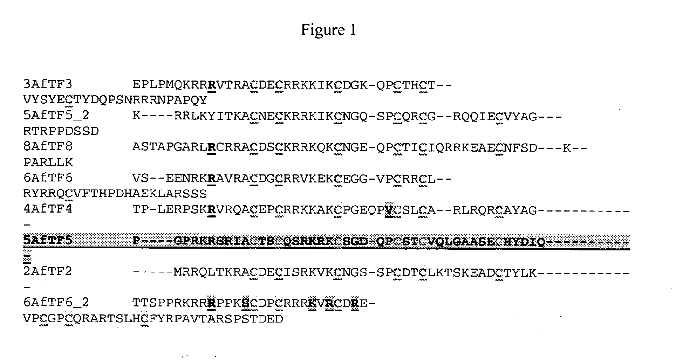

The *Aspergillus fumigatus* genome was surveyed using a *C*. *albicans* tac l p sequence [ABD85289.1]. Eight putative Zn2Cyc6 transcription factors were identified (Figure 1). The putative Zn2Cyc6 transcription factors were sequenced from a triazole susceptible strain and trizole resistant strain of *Aspergillus fumigatus* using a CEQ 8000 capillary electrophoresis DNA sequencer (Beckman coulter, Inc., Fullerton, CA).

The eight putative C6 transcription factors (TFs) were amplified from a well-characterized itraconazole-resistant strain known to over-express ABC transporters. Nascimento et al. Antimicrob. Agents Chemother. (2003) 47:1719-26. The putative mutant TFs were introduced into a wild type recipient strain and transformants screened for resistance to 10 µg/ml itraconazole. Minimum inhibition concentrations (MICs) were determined by the National Committee for Clinical Laboratory Standards (NCCLS) M38-A microdilution method in RPMI 1640 medium in the presence of 0.01 to 16.0 µg/ml after 48 hours of growth at 37°C.

A TF in the mutant strain, named *AzRF1,* located on chromosome 5 (GenBank number XP_753953) was identified that conferred strong resistance to itraconazole (MIC >16 µg/ml) and displayed cross-resistance to voriconazole (MIC > 8.0 µg/ml). *AzRF1* is a C6 transcription factor that is closely related to *Tac1* of *C*. *albicans* with 35% sequence identity to the *Tac1* proteins (GeneBank accession number ABD85289.1).

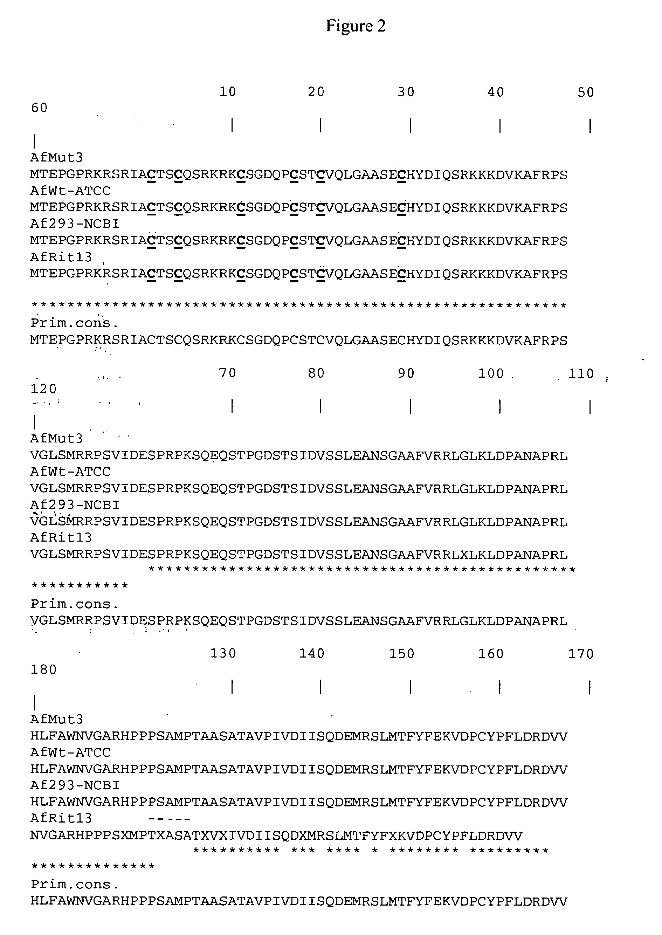

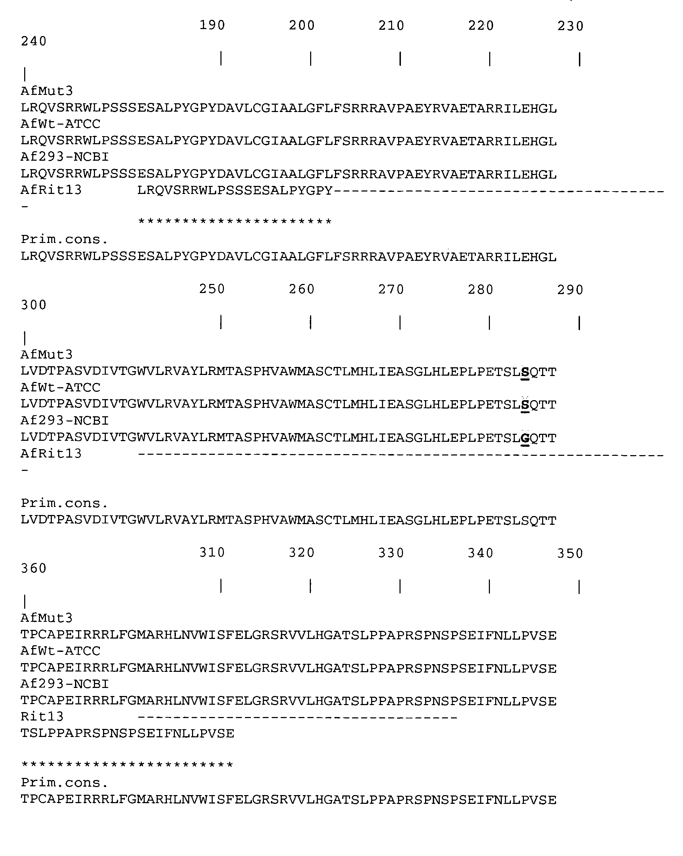

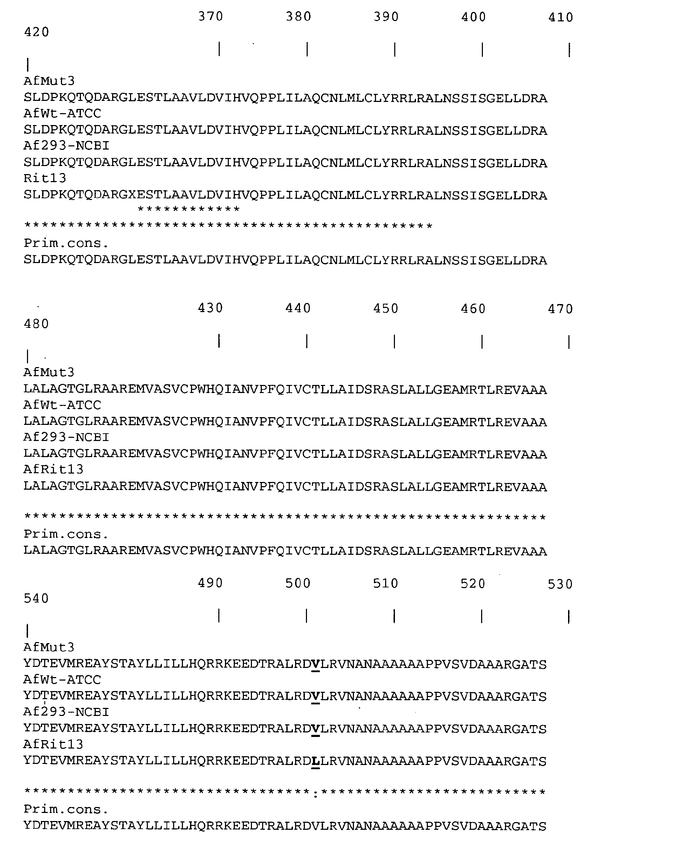

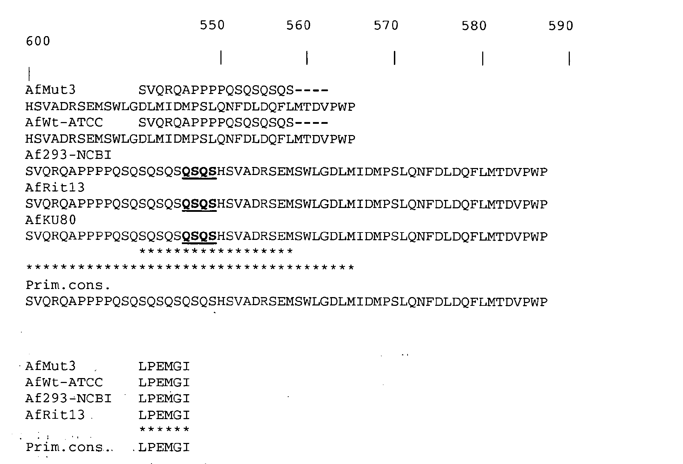

The mutant *AzRF1* contains a deletion of two nucleotide repeats of CTCAGT at position 1675-1686 (Genbank accession number XM_748860), resulting in the loss of four amino acids (QSQS) at position 559-563. See Figure 2.

Introduction of the mutant *AzRF1* allele into *A. fumigatus* wild type strain ATCC 13073 conferred resistance to itraconazole (MIC>16.0 µg/ml) and cross resistance to voriconazole (MIC>8.0 µg/ml) (table 2). To determine whether the triazole resistance displayed by strain *AzRF1-M* was dependent on the presence of the mutant *AzRF1* gene, the AzRF1 PCR product of *A. fumigatus* was cloned into pRG3-AMA1-Bam HI, an autonomous replicating plasmid (Table 1). Plasmid pRG3-AMA1- *AzRF1* was transformed into the triazole susceptible Ku80 strain. The derived mutant *AzRFI-A*, containing the mutant *AzRF1* in an autonomous replicating plasmid was also resistant to triazole drugs (table 2). The loss of the plasmid pRG3-AMA1-*AzRF1* was achieved by 10 rounds of subculturing on drug free minimal medium that resulted in strain *AzRF1-R.* The loss of plasmid pRG3-AMA1- *AzRF1* in strain *AzRF1-R* was confirmed by PCR. Strain *AzRF1-R* had restored sensitivity to triazole drugs (table 2).

*Aspergillus fumigatus* parental strains, derived mutants and plasmids used in this study are described below in Table 1.

**Table 1. Strains and plasmids used in this study**

| **Strain / Plasmid** ATCC 13073 | **Genotype / Description** Wildtype *A. fumigatus* strain | **Source** ATCC |
|---|---|---|
| KY80 DELTA | *A. fumigatus* pyrGAF::Delta KU80; wild-type strain sensitive to echinocandin drugs | da Silva et al. Eukaryot. Cell, 5:207-11(2006). |
| *AzRF1-M* | Homologous recombinant *AzRF1*-mutant of ATCC 13073 formed following transformation with AzRF1 mutant PCR product | This study |
| *AzRF1-H* | Homologous recombinant *AzRF1*-mutant of KU80 formed following transformation with linear pRG3-(pyr4)-AMA1 cut with Bam HI | This study |
| *AzRF1-A* | *AzRF1*-mutant of KU80 transformed with uncut autonomously replicating plasmid pRG3-(pyr4)-*AMA1-AzRF1* ; it also contains wild type *AzRF1* | This study |
| *AzRF1-R* | Strain derived from *AzRF1-*A after plasmid eviction | This study |
| pRG3-AMA1 | Contains *A. nidulans AMA1* and *pyr4* genes | Aleksenko et al. Mol. Microbiol. (1996) 19:565-74; Aleksenko et al. Fungal Genet. Biol. (1997) 21:373-87; and Liu et al. Antimicrob. Agents Chemother. (2004) 48:2490-6 |
| pRG3-AMA1-*AzRF1* | Contains *A. fumigatus fks*1-*AzRF1* mutant in pRG3-(pyr4)-AMA1 | |

**Table 2. Triazole antifungal susceptibilities of parent strain and derived mutants**

| **Strain** | **MIC (µg/ml)** | |
|---|---|---|
| | **ITRACONAZOLE** | **VORICONAZOLE** |
| ATCC 13073 | 0.25 | 0.06 |
| *AzRF1-M* | >16.0 | >8.0 |
| KU80 DELTA | 0.25 | 0.06 |
| *AzRF1-H* | >16.0 | >8.0 |
| *AzRF1-A* | >16.0 | >8.0 |
| *AzRF1*-R | >16.0 | >8.0 |

This data demonstrates that a mutation in *AzRF1* confer resistance to triazole drugs in *A. fumigatus.* This finding may have clinical significance as a marker for triazole resistance in *Aspergillus* infections.

## Claims

1. A method of detecting triazole-resistant Aspergillus in a sample comprising
(A) evaluating said sample for the presence of a gene encoding a AzRF1 transcription factor (SEQ ID NO: 2), wherein said AzRF1 transcription factor contains a mutation of a deletion of residues QSQS at position 559-562 and
(B) correlating the presence of said mutation to the presence of said triazole-resistant Aspergillus.

2. The method of claim 1, wherein the evaluation involves measuring the expression level of said gene.

3. The method of claim 1, wherein said evaluation comprises contacting said sample with an oligonucleotide capable of hybridizing to said mutant gene.

4. The method of claim 3, further comprising amplifying said mutant gene prior to said hybridization.

5. The method of claim 4, wherein said amplification comprises PCR.

6. The method of claim 4, wherein said amplification comprises contacting the sample with primers having the nucleic acid sequences of SEQ ID NO: 3 and SEQ ID NO: 4.

7. The method of claim 3, wherein said oligonucleotide comprises a detectable label.

8. The method of claim 3, wherein said oligonucleotide comprises a fluorophore and a chromophore.

9. The method of claim 3, wherein said oligonucleotide comprises a fluorophore and a non-fluorescent quencher.

10. The method of claim 3 or 4, wherein said oligonucleotide comprises the nucleic acid sequence of SEQ ID NO: 5 or SEQ ID NO: 6.

11. The method of claim 3 or 4, wherein said oligonucleotide comprises greater than 90% homology to the nucleic acid sequence of SEQ ID NO: 5 or SEQ ID NO: 6.

12. The method of claim 3 or 4, wherein said oligonucleotide comprises the nucleic acid sequence of SEQ ID NO: 7, SEQ ID NO:8 or SEQ ID NO: 9.

13. The method of claim 1, wherein the triazole-resistant Aspergillus is Aspergillus fumigatus.

14. The method of claim 1, wherein the triazole-resistant Aspergillus is resistant to itraconazole, posaconazole, voriconazole, isavuconazole, or ravuconazole.

15. A kit for detecting triazole-resistant Aspergillus in a sample comprising an oligonucleotide having the sequence SEQ ID NO: 5 or SEQ ID NO: 6 or a sequence with greater than 90% homology to SEQ ID NO: 5 or SEQ ID NO: 6, wherein said oligonucleotide preferably comprises a label and wherein said kit preferably further comprises one or more amplification primers.

## Patentansprüche

1. Verfahren zum Nachweis von Triazol resistentem Aspergillus in einer Probe umfassend
(A) Bewertung dieser Probe auf das Vorliegen eines für einen AzRF1-Transkriptionsfaktor kodierenden Gens (SEQ ID Nr. 2), worin der AzRF1-Transkriptionsfaktor eine Mutation für eine Deletion der Reste QSQS an Position 559-562 enthält, und
(B) Zuordnung des Vorliegens der Mutation zu dem Vorliegen des Triazol resistenten Aspergillus.

2. Verfahren nach Anspruch 1, worin die Bewertung die Messung des Expressionsspiegels des Gens beinhaltet.

3. Verfahren nach Anspruch 1, worin die Bewertung das in Kontakt bringen der Probe mit einem zur Hybridisierung an das mutierte Gen befähigten Oligonukleotid umfasst.

4. Verfahren nach Anspruch 3, weiterhin umfassend die Amplifizierung des mutierten Gens vor der Hybridisierung.

5. Verfahren nach Anspruch 4, worin die Amplifizierung PCR umfasst.

6. Verfahren nach Anspruch 4, worin die Amplifizierung das in Kontakt bringen der Probe mit Primern umfasst, die die Nukleinsäuresequenzen von SEQ ID Nr. 3 und SEQ ID Nr. 4 haben.

7. Verfahren nach Anspruch 3, worin das Oligonukleotid einen nachweisbaren Marker umfasst.

8. Verfahren nach Anspruch 3, worin das Oligonukleotid ein Fluorophor und ein Chromophor umfasst.

9. Verfahren nach Anspruch 3, worin das Oligonukleotid ein Fluorophor und einen nicht fluoreszierenden Quencher umfasst.

10. Verfahren nach Anspruch 3 oder 4, worin das Oligonukleotid die Nukleinsäuresequenz von SEQ ID Nr. 5 oder SEQ ID Nr. 6 umfasst.

11. Verfahren nach Anspruch 3 oder 4, worin das Oligonukleotid mehr als 90% Homologie zu der Nukleinsäuresequenz von SEQ ID Nr. 5 oder SEQ ID Nr. 6 umfasst.

12. Verfahren nach Anspruch 3 oder 4, worin das Oligonukleotid die Nukleinsäuresequenz von SEQ ID Nr. 7, SEQ ID Nr. 8 oder SEQ ID Nr. 9 umfasst.

13. Verfahren nach Anspruch 1, worin der Triazol resistente Aspergillus *Aspergillus fumigatus* ist.

14. Verfahren nach Anspruch 1, worin der Triazol resistente Aspergillus gegen Itraconazol, Posaconazol, Voriconazol, Isavuconazol oder Ravuconazol resistent ist.

15. Kit zum Nachweis von Triazol resistentem Aspergillus in einer Probe, umfassend ein Oligonukleotid mit der Sequenz von SEQ ID Nr. 5 oder SEQ ID Nr. 6 oder einer Sequenz mit mehr als 90% Homologie zu SEQ ID Nr. 5 oder SEQ ID NR. 6, worin das Oligonukleotid vorzugsweise einen Marker umfasst und worin der Kit vorzugsweise weiterhin einen oder mehrere Amplifzierungsprimer umfasst.

## Revendications

1. Procédé de détection d'une *Aspergillus* résistante aux triazoles dans un échantillon comprenant les étapes consistant à :
(A) évaluer ledit échantillon à la recherche d'un gène codant pour un facteur de transcription AzRF1 (SEQ ID No. : 2), dans laquelle ledit facteur de transcription AzRF1 contient une mutation par délétion des résidus QSQS en position 559 - 562 et
(B) corréler la présence de ladite mutation avec la présence de ladite *Aspergillus* résistante aux triazoles.

2. Procédé selon la revendication 1, dans lequel l'évaluation comprend la mesure du niveau d'expression dudit gène.

3. Procédé selon la revendication 1, dans lequel ladite évaluation comprend la mise en contact dudit échantillon avec un oligonucléotide capable de s'hybrider avec ledit gène mutant.

4. Procédé selon la revendication 3, comprenant en outre l'amplification dudit gène mutant avant ladite hybridation.

5. Procédé selon la revendication 4, dans lequel ladite amplification comprend une PCR.

6. Procédé selon la revendication 4, dans lequel ladite amplification comprend la mise en contact de l'échantillon avec des amorces ayant les séquences d'acide nucléique de SEQ ID No. : 3 et de SEQ ID No. : 4.

7. Procédé selon la revendication 3, dans lequel ledit oligonucléotide comprend un marqueur détectable.

8. Procédé selon la revendication 3, dans lequel ledit oligonucléotide comprend un fluorophore et un chromophore.

9. Procédé selon la revendication 3, dans lequel ledit oligonucléotide comprend un fluorophore et un désactivateur qui n'est pas fluorescent.

10. Procédé selon la revendication 3 ou 4, dans lequel ledit oligonucléotide comprend la séquence d'acide nucléique de SEQ ID No. : 5 ou de SEQ ID No. : 6.

11. Procédé selon la revendication 3 ou 4, dans lequel ledit oligonucléotide comprend une séquence ayant une homologie supérieure à 90 % avec la séquence d'acide nucléique de SEQ ID No. : 5 ou de SEQ ID No. : 6.

12. Procédé selon la revendication 3 ou 4, dans lequel ledit oligonucléotide comprend la séquence d'acide nucléique de SEQ ID No. : 7, de SEQ ID No. : 8 ou de SEQ ID No. : 9.

13. Procédé selon la revendication 1, dans lequel l'*Aspergillus* résistante aux triazoles est l'*Aspergillus fumigatus.*

14. Procédé selon la revendication 1, dans lequel l'*Aspergillus* résistante aux triazoles est résistante à l'itraconazole, au posaconazole, au voriconazole, à l'isavuconazole, ou au ravuconazole.

15. Kit de détection d'une *Aspergillus* résistante aux triazoles dans un échantillon comprenant un oligonucléotide ayant la séquence SEQ ID No. : 5 ou SEQ ID No. : 6 ou une séquence ayant une homologie supérieure à 90 % avec SEQ ID No. : 5 ou SEQ ID No. : 6, dans lequel ledit oligonucléotide comprend de préférence un marqueur et ledit kit comprenant en outre de préférence une ou plusieurs amorces d'amplification.
